# EUROPEAN PATENT SPECIFICATION

(11) **EP 0 363 733 B1**
(45) Date of publication and mention of the grant of the patent: **29.12.1993**
(21) Application number: 89117875.8
(22) Date of filing: 27.09.1989
(51) Int. Cl.: A23K 1/00, A23P 1/04

(54) **Method for preparing mixtures for human and animal feed use containing uniformly distributed active substances**
Verfahren zur Herstellung von Mischungen, verwendbar in Nahrungsmitteln und Tierfutter, die gleichmässig verteilte wirksame Substanzen enthalten
Procédé de préparation de mélanges destinés à être utilisés dans de produits alimentaires contenant des substances actives, uniformément réparties

(30) Priority: 14.10.1988 IT 2231188
(43) Date of publication of application: 18.04.1990
(73) Proprietor: DOX-AL ITALIA S.p.A., I-20050 Correzzana Milano (IT)
(72) Inventor: Grabitz, Ernst B., I-22064 Casatenovo (COMO) (IT)
(74) Representative: Gervasi, Gemma, Dr.

(56) References cited:
- EP-A- 0 165 577
- EP-A- 0 217 631
- EP-A- 0 272 119
- FR-A- 2 361 935
- FR-A- 2 368 899
- FR-A- 2 447 748
- GB-A- 1 120 775
- GB-A- 2 016 043
- US-A- 3 495 988
- Chambers 20th Century Dictionary New edition 1983,page 482
- Food Technology, vol 27,no 11, 1983, pages 24-44, Chicago, Us; J.A. Bakan et al,: "Microencapsulation of food and related products" * Pages 28-40 under "Core and coation materials" *.

## Description

### Field of the invention

This invention relates to a method for preparing mixtures for human and animal feed use comprising active substances, and to the mixtures obtained.

### Prior art

It is known to prepare flour or meal mixtures containing active substances for animal use, in which the active substances are present in small quantities in all cases of less than 10% by weight.

The preparation of said mixtures involves various difficulties. Firstly, the mixtures are never homogeneous because the small active substance quantities cannot be dispersed uniformly in the large flour or meal mass because of differences in particle size, specific gravity and hydrophilic-lipophilic characteristics.

The same difficulties are also encountered in the preparation of premixes containing large active substance quantities.

Moreover, if in preparing a premix or the final product the actual mixing operations are extended in order to obtain a product which is as homogeneous as possible, there is an increased risk that the active substances will degrade and interact with the other mixture components.

To overcome these drawbacks, the prior art uses active substance overdosing in order to compensate the degradation, the alteration caused by interaction, and the non-uniform distribution of the active substance.

French patent 2.368.899 discloses a method for encapsulating the particles of the active substance (Nitrovin) thus obtaining the active substance in a stable form: this method does not offer a good solution of the problem to distribute uniformly a very little amount of active substance in the whole mass of the feed.

GB patent 2.016.043 proposes a method wherein the active substance is encapsulated in a large excess of an hydrophilic material. The use of a large amount of the encapsulating material (Carbowax, sugar) not so cheap as many raw materials used in the fodder production, involves higher cost of the final product.

French patent 2.361.935 uses greasy-oily encapsulating substances which involve the use of organic solvent: consequently the method is not cheap.

### Summary of the invention

The drawbacks of the prior art are obviated by the method for preparing mixtures for human and animal feed use comprising active substances, according to the present invention, which is characterised by:
a) applying the active substance to a water-insoluble granular support through mixing in a powder mixer the ingredients in presence of coadiuvant agents selected from: Ca or Mg salt of fatty acids, vegetable or animal or mineral oil, said coadiuvant agents being in amount between 5% and 20% by w. with respect to the support-active substance mixture, and/or in presence of non-ionic surface active agent in amount between 0.5 and 10% by w.;
b) applying to the granular material coming from step a) a coating layer of polymeric hydrophilic substance soluble or dispersible in water, selected from: cellulose derivatives (methylcellulose, hydroxypropylcellulose, methylhydroxypropylcellulose, carboxymethylcellulose, ethylcellulose and acetylcellulose), vinyl polymer (polyvinylpyrrolidone, polyvinylalcohol, polyvinylacetate, copolymer vinylacetate/ethylene), arabic gum, polyethyleneglycols, higher fatty acids and alcohols, hydrogenated fatty substances, said coating material being applied in form of aqueous solution or dispersion, the amount of the polymerioc coating material being between 2 and 15% by w. with respect to the granular material to be coated;
c) mixing the coated granular product of step b) with flours or meals for human or animal feed use to obtain a final product with the desired active substance content.

By operating in this manner a product with uniform distribution of the active substance is obtained.

### Detailed description of the invention

The characteristics and advantages of the method for preparing mixtures for human and animal feed use comprising active substances, according to the present invention, will be more apparent from the following detailed description.

A granular material is firstly prepared to act as a support for the active substance. This material can be of mineral or vegetable origin, its characteristics being chosen according to the desired specific gravity of the final product and active substance release time.

Particularly suitable materials of mineral origin for animal use are vermiculite and calcium carbonate with a particle size distribution of 90% less than 0.88 mm (20 mesh).

Suitable materials of vegetable origin are maize granulates, cereal flakes, cereal extrusions and carob granulates. Maize cobs ground to a particle size distribution of 85% less than 0.54 mm (30 mesh) are particularly suitable. The most varied active substances used for human and animal feed purposes can be employed in the mixtures, comprising vitamins, amino acids, microelements such as copper, cobalt, iron, zinc, manganese, molybdenum and others, and pharmaceutically active substances such as growth promoters, antibiotics, sulphamidics, antiparasitics and substances of therapeutic and/or preventive action in general.

Antioxidants and/or stabilizers are added to the active substances when necessary. Polyvinylpyrrolidone or Ca or Mg salts of fatty acids can for example be added.

The active substance is applied to the support by mixing in a solids mixer. The quantity of applied active substance is between 1 and 45% by weight with respect to the support.

In order to facilitate adhesion of the active substance and its penetration into the support, the application of the active substance can be preceded by mixing the support with the Ca or Mg salts of fatty acid mixtures and with a vegetable, animal or mineral oil in a quantity of between 5 and 20% by weight with respect to the support-active substance mixture.

Preferred examples of said oils are soybean oil, castor oil and paraffin oil.

To further improve adhesion of the active substance to the support, the mixture consisting of the support, the active substance and possibly the other said substances is mixed with a surface active agent in a quantity of between 0.5 and 10% with respect to said mixture.

The choice of surface active agent is based on the characteristics of the support material. Suitable surface active agents are the physiologically compatible non-ionic surface agents such as monoesters of propyleneglycol and of the food fatty acids, stearyl-2-lactylic acid, acetic, lactic, citric, tartaric and monoacetyltartaric esters of the mono and diglycerides of food fatty acids, glycerin polyethyleneglycol ricinoleate, polyethyleneglycol esters of soybean oil fatty acids, sorbitan monostearate, sorbitan tristearate, sorbitan monolaurate, sorbitan monooleate, sorbitan monopalmitate and propyleneglycol alginate.

Mixtures of these surface active agents with polyethyleneglycol and/or with propyleneglycol and/or with glycerin can also be used.

The surface active agent is applied by spraying it onto the solid mixture while this is kept under mixing.

The product granules obtained in this manner are coated with a thin layer of water-soluble or water-dispersable non-toxic polymer which forms a film at a temperature less than 60°C.

With this type of polymer there is the great advantage of operating without organic solvents and only with water at low temperature, and thus without the risk of alteration to the active substance.

The active substance thus remains enclosed within the coated granule and has no possibility of separating from the support and coming into contact with other components of the mixture during the subsequent mixing, bagging, transportation and other operations.

Polymers suitable for said coating are for example cellulose derivatives (methylcellulose, hydroxypropylcellulose, methylhydroxypropylcellulose, carboxymethylcellulose, ethylcellulose and acetylcellulose), vinyl polymers (polyvinylpyrrolidone, polyvinyl alcohol and polyvinylacetate), gum arabic, substances of wax type such as polyethyleneglycols, higher alcohols, higher fatty acids and hydrogenated fatty substances.

After oral administration these polymers dissolve or disintegrate in the liquids of the oral cavity, stomach or intestine.

The choice from said polymers is made on the basis of the hydrophilic and/or lipophilic characteristics of the mass into which the active substance is to be distributed, and on the basis of the desired active substance release characteristics.

The polymer is added to the support-active substance mixture in a quantity of between 2 and 15% by weight with respect to the mixture.

The application is made by adding a solution or dispersion of the polymer in water in the weight ratio of between 1:0.5 and 1:10 to the support-active substance mixture while this latter is kept under mixing.

The granular product coated with polymer as described is heated by hot air to a temperature of between 25 and 70°C to reduce its water content to between 2 and 10% by weight.

The product obtained in this manner is finally mixed with flour or meal for human or animal feed use to obtain a final product containing between 0.05 and 5% of active substance by weight.

The distribution of the active substance within the final product is uniform.

The following examples are given for illustrative purposes.

### EXAMPLE 1

68.6 g of maize cob ground to a particle size distribution of 85% less than 0.54 mm (30 mesh) and 10.0 g of Ca salts of fatty acids are loaded into a laboratory solids mixer and mixed for 5 minutes.

16.4 g of a mixture of vitamins A, D, E, B₁, B₂ and B₁₂ and polyvinyl pyrrolidone are fed in slowly while mixing, and mixing is then continued for a further 5 minutes.

1 g of glycerin polyethyleneglycol ricinoleate (a non-ionic surface active agent) is slowly added while mixing, and mixing is continued for a further 5 minutes to obtain the mixture A.

A solution of 1.0 g of vinylacetate-ethylene copolymer and 3.0 g of polyvinyl alcohol in 16 g of water is prepared separately.

This solution is added slowly to mixture A while mixing, and mixing is continued for a further 5 minutes.

The mixture is heated for 30 minutes with hot air at a temperature of 60°C while mixing, such that the temperature of the exit air does not exceed 30°C.

The mixture is then cooled for 45 minutes with air at ambient temperature while mixing.

The granular product obtained is mixed with maize flour of 0.54 mm (30 mesh) particle size in such a quantity as to obtain a vitamin content of 1.6% by weight.

The final product has a uniform vitamin distribution.

### EXAMPLE 2

Example 1 is repeated in an industrial mixer using the following raw material quantities:

| | |
|---|---|
| ground maize cobs | 200.0 kg |
| Ca salts of fatty acids | 10.0 kg |
| soybean oil | 29.7 kg |
| glycerin polyethyleneglycol ricinoleate | 6.0 kg |
| vitamins | 49.6 kg |
| polyvinyl alcohol | 2.4 kg |
| vinyl acetate polymers | 12.0 kg |
| water | 9.6 kg |

The final product has the same uniform active substance distribution characteristics as in Example 1.

### EXAMPLE 3

60.0 g of granular calcium carbonate having a particle size distribution of 90% less than 0.88 mm (20 mesh) and 10.0 g of soybean oil are loaded into a laboratory solids mixer and mixed for 5 minutes.

25.5 g of powdered NITROVIN (growth promoter) are fed in slowly while mixing, and mixing is then continued for a further 5 minutes.

2 g of Tween 20 (a non-ionic surface active agent) are slowly added while mixing, and mixing is continued for a further 5 minutes to obtain the mixture B.

A solution of 2.5 g of polyvinyl alcohol in 10 g of water is prepared separately, this solution is added slowly to mixture B while mixing, and mixing is continued for a further 5 minutes.

The mixture is heated for 30 minutes with hot air at a temperature of 60°C while mixing, and then cooled for 45 minutes with air at ambient temperature always while mixing.

The product obtained is mixed with maize flour in such a quantity as to obtain an active substance content of 2.5% by weight, to obtain a final product of uniform composition.

### EXAMPLE 4

Example 3 is repeated using the following raw materials:

| | |
|---|---|
| calcium carbonate | 80.7 g |
| Ca salts of fatty acids | 6.0 g |
| microelements (soluble Cu, Fe, Zn, Mn salts) | 10.0 g |
| Tween 20 | 0.3 g |
| Polyethyleneglycol | 3.0 g |

The final product has homogeneous characteristics.

### EXAMPLE 5

Example 3 is repeated in a mixer of semi-industrial type using the following raw materials:

| | |
|---|---|
| calcium carbonate | 88.52 kg |
| vitamin A | 1.25 kg |
| vitamin D | 0.25 kg |
| vitamin E | 1.50 kg |
| vitamin B₁ | 0.10 kg |
| vitamin B₂ | 0.30 kg |
| vitamin B₁₂ | 2.00 kg |
| vitamin K₃ | 0.10 kg |
| nicotinamide | 1.00 kg |
| calcium pantothenate | 1.78 kg |
| polyvinyl alcohol | 3.20 kg |
| water | 12.80 kg |

The final product has uniform vitamin distribution.

## Claims

1. A method for preparing mixtures for human and animal feed use, comprising active substances, characterized by:
a) applying the active substance to a water-insoluble granular support through mixing in a powder mixer the ingredients in presence of coadiuvant agents selected from: Ca or Mg salt of fatty acids, vegetable or animal or mineral oil, said coadiuvant agents being in amount between 5% and 20% by w. with respect to the support-active substance mixture, and/or in presence of non-ionic surface active agent in amount between 0.5 and 10% by w.;
b) applying to the granular material coming from step a) a coating layer of polymeric hydrophilic substance soluble or dispersible in water, selected from: cellulose derivatives (methylcellulose, hydroxypropylcellulose, methylhydroxypropylcellulose, carboxymethylcellulose, ethylcellulose and acetylcellulose), vinyl polymer (polyvinylpyrrolidone, polyvinylalcohol, polyvinylacetate, copolymer vinylacetate/ethylene), arabic gum, polyethyleneglycols, higher fatty acids and alcohols, hydrogenated fatty substances, said coating material being applied in form of aqueous solution or dispersion, the amount of the polymeric coating material being between 2 and 15% by w. with respect to the granular material to be coated;
c) mixing the coated granular product of step b) with flours or meals for human or animal feed use to obtain a final product with the desired active substance content.

2. A method as claimed in claim 1, characterized in that said support consists of a material of mineral origin.

3. A method as claimed in claim 1, characterized in that said support consists of a material of vegetable origin.

4. A method as claimed in claim 1, characterized in that said support consists of maize cob granulates, cereal flakes, cereal extrusions or carob granulates.

5. Method as claimed in claim 1, characterized in that said oil is soybean oil, castor oil or paraffin oil.

6. A method as claimed in claim 1, characterized in that said surface active agents are monoesters of propyleneglycol and of the food fatty acids, acetic, lactic, citric, tartaric and monoacetyltartaric esters of the mono and diglycerides of food fatty acids, glycerin polyethyleneglycol ricinoleate, polyethyleneglycol esters of soybean oil fatty acids, sorbitan monostearate, sorbitan tristearate, sorbitan monolaurate, sorbitan monooleate, sorbitan monopalmitate, propyleneglycol alginate and their mixtures with polyethyleneglycol and/or with propyleneglycol and/or with glycerin.

## Patentansprüche

1. Verfahren zur Herstellung von Mischungen, die in Nahrungsmitteln und Tierfutter verwendbar sind und die wirksame Substanzen umfassen, gekennzeichnet durch:
a) Aufbringen der Wirksubstanz auf einen wasserunlöslichen körnigen Träger durch Mischen der Inhaltsstoffe in einem Pulvermischer in Gegenwart von Hilfsstoffen, ausgewählt aus: dem Ca- oder Mg-Salz von Fettsäuren, pflanzlichem oder tierischem Öl oder Mineralöl, wobei diese Hilfsstoffe in einer Menge zwischen 5 und 20 Gew.-%, bezogen auf die Träger-Wirksubstanzmischung, und/oder in Gegenwart eines nichtionischen oberflächenaktiven Mittels in einer Menge zwischen 0,5 und 10 Gew.-% vorliegen;
b) Aufbringen einer Überzugsschicht eines polymeren hydrophilen Stoffes, der in Wasser löslich oder dispergierbar ist, ausgewählt aus: Cellulosederivaten (Methylcellulose, Hydroxypropylcellulose, Methylhydroxypropylcellulose, Carboxymethylcellulose, Ethylcellulose und Acetylcellulose), Vinylpolymeren (Polyvinylpyrrolidon, Polyvinylalkohol, Polyvinylacetat, ein Copolymer aus Vinylacetat und Ethylen), Gummiarabikum, Polyethylenglykolen, höheren Fettsäuren und Alkoholen, hydrierten fettartigen Stoffen, auf das von Schritt a) kommende körnige Material, wobei das Überzugsmaterial in Form einer wäßrigen Lösung oder Dispersion aufgetragen wird und die Menge des polymeren Überzugsmaterials zwischen 2 und 15 Gew.-%, bezogen auf das zu beschichtende körnige Material, beträgt;
c) Mischen des beschichteten körnigen Produkts von Schritt b) mit Mehlen oder Grobmehlen zur Verwendung in menschlicher oder tierischer Nahrung, wodurch ein Endprodukt mit dem gewünschten Gehalt an wirksamer Substanz erhalten wird.

2. Verfahren gemäß Anspruch 1, dadurch gekennzeichnet, daß der Träger aus einem Material mineralischen Ursprungs besteht.

3. Verfahren gemäß Anspruch 1, dadurch gekennzeichnet, daß der Träger aus einem Material pflanzlichen Ursprungs besteht.

4. Verfahren gemäß Anspruch 1, dadurch gekennzeichnet, daß der Träger aus Maiskolbengranulat, Getreideflocken, Getreideextrudaten oder Johannisbrotgranulaten besteht.

5. Verfahren gemäß Anspruch 1, dadurch gekennzeichnet, daß das Öl Sojabohnenöl, Rizinusöl oder Paraffinöl ist.

6. Verfahren gemäß Anspruch 1, dadurch gekennzeichnet, daß die oberflächenaktiven Mittel Monoester von Propylenglykol und von den eßbaren Fettsäuren, Essigsäure-, Milchsäure-, Zitronensäure-, Weinsäure- und Monoacetylweinsäureester der Mono- und Diglyceride der eßbaren Fettsäuren, Glycerinpolyethylenglykolricinoleat, Polyethylenglykolester von Sojabohnenölfettsäuren, Sorbitanmonostearat, Sorbitantristearat, Sorbitanmonolaurat, Sorbitanmonooleat, Sorbitanmonopalmitat, Propylenglykolalginat und deren Mischungen mit Polyethylenglykol und/oder mit Propylenglykol und/oder mit Glycerin sind.

## Revendications

1. Procédé pour la préparation de mélanges destinés à des produits alimentaires pour l'homme et l'animal, comprenant des substances actives, caractérisé par les étapes consistant à :
a) appliquer la substance active sur un support granulaire insoluble dans l'eau par mélange dans un mélangeur de poudre les ingrédients en présence d'agents coadjuvants choisis parmi : sel de Mg ou Ca des acides gras, huile végétale, animale ou minérale, les agents coadjuvants étant dans une quantité entre 5 % et 20 % en poids par rapport au mélange de substance active de support, et/ou en présence d'agent tensioactif non ionique dans une quantité entre 0,5 et 10 % en poids ;
b) appliquer au matériau granulaire provenant de l'étape a) une couche de revêtement de substance hydrophile polymère soluble ou pouvant être dispersée dans l'eau, choisie parmi: les dérivés de la cellulose (méthylcellulose, hydroxypropylcellulose, méthylhydroxypropylcellulose, carboxyméthylcellulose, éthylcellulose et acétylcellulose), polymères vinyliques (polyvinylpyrrolidone, polyvinylalcool, polyvinylacétate, vinylacétate/éthylène de copolymère), gomme arabique, polyéthylèneglycols, acides gras supérieurs et alcools, substances grasses hydrogénées, ce matériau de revêtement est appliqué sous forme de solution aqueuse ou de dispersion, la quantité du matériau de revêtement polymère se situe entre 2 et 15 % en poids par rapport au matériau granulaire à revêtir ;
c) mélanger le produit granulaire revêtu de l'étape b) avec des farines pour produits alimentaires pour l'homme ou les animaux et obtenir un produit final possédant la teneur en substance active souhaitée.

2. Procédé selon la revendication 1, caractérisé en ce que le support consiste en un matériau d'origine minérale.

3. Procédé selon la revendication 1, caractérisé en ce que le support consiste en un matériau d'origine végétale.

4. Procédé selon la revendication 1, caractérisé en ce que le support consiste en des granulats d'épis de maïs, de flocons de céréale, d'extrusions de céréale ou de granulats de caroube.

5. Procédé selon la revendication 1, caractérisé en ce que l'huile est de l'huile de soja, de l'huile de ricin ou de l'huile de paraffine.

6. Procédé selon la revendication 1, caractérisé en ce que les agents tensioactifs sont des monoesters de propylèneglycol et des acides gras pour produit alimentaire, l'acide acétique, lactique, citrique, tartarique et les esters monoacétyltartariques des mono- et diglycérides des acides gras alimentaires, le ricinoléate de polyéthylèneglycol de glycérine, les esters de polyéthylèneglycol des acides gras de l'huile de soja, le monostéarate de sorbitanne, le tristéarate de sorbitanne, le monolaurate de sorbitanne, le monooléate de sorbitanne, le monopalmitate de sorbitanne, l'alginate de propylèneglycol et leurs mélanges avec le polyéthylèneglycol et/ou avec le propylèneglycol et/ou la glycérine.
